# EUROPEAN PATENT SPECIFICATION

(11) **EP 1 569 657 B1**
(45) Date of publication and mention of the grant of the patent: **20.04.2011**
(21) Application number: 03782263.2
(22) Date of filing: 01.12.2003
(51) Int. Cl.: A61K 31/685, A61K 31/688, A61K 31/70, A61P 35/04

(54) **USE OF AMPHIPHILIC LIPIDS FOR THE PREPARATION OF A PHARMACEUTICAL COMPOSITION FOR REDUCING TUMOR METASTASIS**
VERWENDUNG VON AMPHIPHILEN LIPIDEN ZUR HERSTELLUNG EINER PHARMAZEUTISCHEN ZUSAMMENSETZUNG ZUR HEMMUNG VON TUMOR METASTASEN
UTILISATION DE LIPIDES AMPHIPHILIQUES DANS LA PREPARATION D'UNE COMPOSITION PHARMACEUTIQUE DESTINEE A DIMINUER UNE METASTASE TUMORALE

(30) Priority: 03.12.2002 EP 02026897
(43) Date of publication of application: 07.09.2005
(73) Proprietor: Fresenius Kabi Deutschland GmbH, 61352 Bad Homburg v.d.H. (DE)
(72) Inventor: SCHLOTZER, Ewald, 61440 Oberursel (DE); WÄRNHEIM, Torbjörn, S-163 42 Spanga (SE); JANSEN, Marc, 52074 Aachen (DE); TREUTNER, Karl-Heinz, 10114 Berlin (DE)
(74) Representative: UEXKÜLL & STOLBERG
(86) International application number: PCT/EP2003/013507
(87) International publication number: WO 2004/050097

(56) References cited:
- EP-A- 0 381 514
- WO-A1-99/51244
- US-A- 4 797 479
- HAKOMORI SEN-ITIROH ET AL: "Glycosphingolipid antigens and cancer therapy." CHEMISTRY & BIOLOGY (LONDON), vol. 4, no. 2, 1997, pages 97-104, XP009010432 ISSN: 1074-5521
- SAKAI ATSUSHI ET AL: "Deacylation-reacylation cycle: A possible absorption mechanism for the novel lymphotropic antitumor agent dipalmitoylphosphatidylfluorourid ine in rats." JOURNAL OF PHARMACEUTICAL SCIENCES, vol. 82, no. 6, 1993, pages 575-578, XP002240546 ISSN: 0022-3549
- KILLION J J ET AL: "Systemic targeting of liposome-encapsulated immunomodulators to macrophages for treatment of cancer metastasis." IMMUNOMETHODS. UNITED STATES JUN 1994, vol. 4, no. 3, June 1994 (1994-06), pages 273-279, XP002240547 ISSN: 1058-6687
- TALSMA H ET AL: "THE SIZE REDUCTION OF LIPOSOMES WITH A HIGH PRESSURE HOMOGENIZER MICROFLUIDIZER CHARACTERIZATION OF PREPARED DISPERSIONS AND COMPARISON WITH CONVENTIONAL METHODS" DRUG DEVELOPMENT AND INDUSTRIAL PHARMACY, vol. 15, no. 2, 1989, pages 197-208, XP009010461 ISSN: 0363-9045
- DATABASE MEDLINE [Online] US NATIONAL LIBRARY OF MEDICINE (NLM), BETHESDA, MD, US 18 April 1987 'Ten centre trial of artificial surfactant (artificial lung expanding compound) in very premature babies. Ten Centre Study Group.' Database accession no. NLM2890398 & BRITISH MEDICAL JOURNAL (CLINICAL RESEARCH ED.) 18 APR 1987 LNKD- PUBMED:2890398, vol. 294, no. 6578, 18 April 1987 (1987-04-18), pages 991-996, ISSN: 0267-0623

## Description

The present invention relates to the use of amphiphilic lipids as the only active compound for the preparation of a pharmaceutical composition for reducing tumor metastasis, wherein the amphiphilic lipids are phospholipids.

Cancerous dieseases and tumors in general are among the major causes for human deaths and severe illness. Even after surgical removal of a tumor, patients frequently suffer from tumor disease, mostly from tumor metastases. The development of peritoneal carcinosis after surgical treatment of digestive cancers for example still is a frequent cause of recurrence. In gastric cancer with serosal invasion up to 50% of patients develop peritoneal carcinosis even if curative resection is performed (Boku T et al., Br J Surg 1990; 77:436-9; and Jansen M et al., Chirurg 2001; 72:561-5). Several studies demonstrated that detection of free, isolated tumor cells in the peritoneal cavity can serve as a prognostic marker for postoperative survival of colorectal and gastric cancer patients (Koga S et al., J Cancer Res Clin Oncol 1984; 108:236-8; Schott A et al., Ann Surg 1998; 227:372-9; Bando E et al., Am J Surg 1999; 178:256-62; and Broll R et al., Langenbecks Arch Chir 1996; 381:51-8).

Peritoneal tumor recurrence may be a result of residual, intraperitoneal free tumor cells caused by serosal invasion of the primary tumor or intraoperative exfoliation. The invasion of tumor cells into the peritoneal wall is a complex process including attachment, proteolysis and migration (Schwartz OK, Semin Oncol 1996; 23:316-24). The most important step in developing peritoneal dissemination seems to reside in the adhesion of tumor cells to mesothelial cells or extracellular matrix components (Kiyasu Y et al., Cancer Res 1981; 41:1236-9; Koga S et al., Gann 1980; 71:8-13; and Schwartz OK, Semin Oncol 1996; 23:316-24). Experimental studies suggest that peritoneal metastases primarily tend to occur at sites of injured peritoneum (Yashiro M et al., Cancer 1996; 77:1668-75).

Attachment of gastric cancer cells to the peritoneal surface represents the first step necessary in tumor cell invasion (Schwartz OK, Semin Oncol 1996; 23:316-24). In a scanning electron microscopic study Kiyasii et al. could demonstrate that cancer cells did not adhere to the mesothelial cells but to the free lying submesothelial connective tissue. The mesothelial cells, however, became hemispherical and exfoliation occurred in the presence of gastric cancer cells (Kiyasu Y et al., Cancer Res 1981; 41:1236-9).

Since surgery alone remains an incomplete cure, numerous additional treatments have been evaluated. Intraperitoneal chemotherapy is a reliable method for treating peritoneal seeding, but frequent complications have been described (Hagiwara et al., Surgery 1988; 104:874-881; Fass et al., Langenbecks Arch Chir Suppl Kongress bd 1998; 115:1363-1366; Sayag et al., Oncology 1993; 50:333-337; Fujimura et al., Int Surg 1999; 84:60-66; Fujimoto et al., Ann Surg 1990; 212:592-596; and Koga et al., Cancer 1988; 61:232-237).

Attempts were made to inhibit tumor cell attachment. Since the peritoneal tumor cell adhesion is mediated by adhesion molecules, antibodies with specificity for adhesion molecules were used to inhibit dissemination of human gastric cancer cells (Chailley-Heu B et al., Biochem J 1997; 328(Pt1):251-6). The main problem for the clinical use of respective antibodies seems to be the different expression of these antigens in tumors of different origin and other cell lines.

Further, tests to reduce peritoneal metastases using dextran sulfate and sodium hyaluronate in animal models have been reported (Hagiwara A et al., Anticancer Drugs 1997; 8:894-7; and Haverlag R et al., Eur J Surg 1999; 165:791-5).

Dextran sulfate led to a reduced tumor implantation at the sites of injury in the abdominal wall in mice (Hagiwara A et al., Anticancer Drugs 1997; 8:894-7; and Hagiwara A et al., Anticancer Drugs 2000; 11:873-7). At the same time the authors reported a prolonged survival in the treatment group after inoculation of melanoma cells. However, several side effects were observed when using dextran for adhesion prevention. The main problems were edema, pleura effusion, life-threatening malfunction of coagulation and severe allergic reactions (diZerega GS, Curr Opin Obstet Gynecol 1996; 8:230-7; and Treutner KH et al., Chirurg 2000; 71:510-7).

Sodium hyaluronate proved to enhance colorectal tumor cell metastatic potential, probably mediated by the CD44 receptor (Tan et al., Br J Surg 2001; 88:246-250). It was thus concluded that this treatment leads to exponentiation of intraperitoneal tumor growth.

EP-A-0 381 514 and HAKOMORI SEN-ITIROH ET AL ("Glycosphingolipid antigens and cancer therapy." CHEMISTRY & BIOLOGY (LONDON), vol. 4, no. 2, 1997, pages 97-10) concern the use of sphingolipids and glycosphingolipids for reducing tumor metastasis. US-A-4 797 479 and SAKAI ATSUSHI ET AL ("Deacylation-reacylation cycle: A possible absorption mechanism for the novel lymphotropic antitumor agent dipalmitoylphosphatidylfluorourid ine in rats." JOURNAL OF PHARMACEUTICAL SCIENCES, vol. 82, no. 6, 1993, pages 575-578) disclose the use of nucleoside-phospholipids as anti-tumor and anti metastatic agents.

In view of the above prior art, the present invention relates to the problem of medical treatment of cancerous diseases, specifically to the inhibition of tumor recurrence and metastases.

According to the present invention this problem is solved by the use of amphiphilic lipids as the only active compound for the preparation of a pharmaceutical composition for reducing tumor metastasis, wherein the amphiphilic lipids are phospholipids.

The present inventors have surprisingly shown that tumor metastases can significantly be reduced by administration of an amphiphilic lipid, wherein the amphiphilic lipid is a phospholipid, which results in an increase of the survival rates. The amphiphilic lipid is safe and causes no undesired side effects.

The term "amphiphilic lipid" is used in this application to refer to an organic molecule with a glycerol (or polyol, such as alcohol) backbone substituted with two acyl chains coupled via ester bonds and a polar group substituted to the remaining hydroxyl group of the alcohol. The term amphiphilic clarifies that respective compounds contain both hydrophilic and lipophilic groups at the same time. The amphiphilic lipids to be used in accordance with the present invention form bilayers, preferably liposomes, upon dispersion in a liquid. For the medical use of the present invention it is necessary that the amphiphilic lipids are pharmaceutically acceptable. Any amphiphilic lipid which is a phospholipid and meets these requirements can be used in accordance with the present invention.

The term "pharmaceutical composition" is used in the present application to refer amongst others to pharmaceutical compositions which have to be approved by a regulatory authority (EMEA, FDA, etc.). However, the term "pharmaceutical composition" is not limited to these compositions but encompasses any composition or medical device which contains amphiphilic lipids as the only active compound and is suitable for the claimed medical use.

The present disclosure encompasses the use of a single specific type of amphiphilic lipid for the preparation of a pharmaceutical composition for reducing tumor metastasis as well as the use of a combination of various amphiphilic lipids for this purpose, wherein the amphiphilic lipid(s) is /are (a) phospholipid(s). More specifically, the present disclosure includes the medical use of known phospholipids for reducing tumor metastasis which have sofar been used as emulsifiers for the preparation of fett emulsions for parenteral nutrition.

In the present application, the term phospholipid (PL) is used to refer to a glycerol backbone containing two acyl chains coupled via ester bonds and a polar, phosphorous containing group substituted to the remaining hydroxyl group of the glycerol. Respective polar lipid derivatives can be of natural, semi-synthetic or synthetic origin. Phospholipids can thus be provided by and be prepared from natural sources, since they are a common membrane component in cells of plant and animal origin. The naturally occuring phospholipids can be extracted and purified. Respective phospholipids have sofar for example been used for the preparation of fett emulsions for parenteral nutrition.

There are several different sources of pharmaceutical grade phospholipids, e.g. egg and soybean. Depending on their origin, the detailed composition in terms of fatty acid composition as well as polar group composition will vary. Phospholipids of more defined molecular composition can be provided by a combination of purification and fractionation of endogenous phospholipids including a final synthetic step (semisynthetic phospholipids) or by a purely synthetic route (synthetic phospholipids).

Lipids and more specifically phospholipids are natural components of the abdominal cavity. It was speculated that this substance may form a lubricant layer on the peritoneal surface (Beavis et al., Perit Dial Int 1994; 14:348-355; and Chailley-Heu et al., Biochem J 1997; 328(Pt1):251-256). Intraperitoneal application of phospholipids led to a significant decrease of adhesion formation, especially at sites of peritoneal injury, without any side effect on healing of anastomoses, laparotomy wounds, and liver incisions (Muller et al., J Surg Res 2001; 96:68-74; Muller et al., Langenbecks Arch Surg 2001; 386:278-284; and WO 98/535800). As of today, however, lipids as such, have not been suggested as active substance for treatment of cancerous diseases, in particular for reduction of metastasis.

In the medical use of amphiphilic lipids according to the present disclosure tumor metastases are reduced amongst others due to inhibition of tumor cell adhesion, inhibition of adhesion of metastastatic cells and/or inhibition of tumor metastasis growth.

In accordance with the present disclosure the amphiphilic lipids can be used for reduction of any tumor metastasis, such as recurrence of peritoneal carcinosis and various other cancers, for example, gastric, colon, ovarian or pancreatic cancer.

The medical use of the present disclosure aims, of course, at reducing the occurance of metastases to zero. However, a medical use of amphiphilic lipids which achieves a significant reduction of the number of metastases and/or an increase in the survival rate already has to be considered as a significant improvement and is covered by the present invention.

The amphiphilic lipids can be administered for prophylactic treatment. According to a specifically preferred embodiment, the amphiphilic lipids are used for prophylactic treatment of intraperitoneal recurrence of tumors, specifically malignant tumors, e.g. gastro-intestinal cancer and cancer of the genito-urinary system such as ovarian cancer or bladder cancer. The patient may have undergone curative resection before treatment or be subject to a respective surgical treatment shortly after administration of amphiphilic lipids.

In the medical use of the present invention, the amphiphilic lipid which is a phospholipid is present as the only active compound for reducing tumor metastasis in the pharmaceutical composition. The term "active compound" is used in the present application to refer to that compound in a composition which is responsible for the observed effect (reduction of tumor metastasis). Specific advantages are achieved with the amphiphilic lipid which is a phospholipid as the only active compound, since amphiphilic lipids do not cause any side effects in contrast to the majority of anti-tumor compounds and radioactive drugs, which are all known cause significant side effects.

In one aspect of the present disclosure the amphiphilic lipids are used to treat a mammal. The treatment of humans is especially preferred.

The amphiphilic lipids can be administered in any form and concentration which is clinically effective. According to one embodiment of the present invention a phospholipid is dispersed an aqueous solution. The solution preferably contains the phospholipid in a concentration of 0.05% to 25% (w/v), more preferably in a concentration of 0.5% to 20% (weight/weigt) and most preferably in a concentration of 1,5% to 10% (weight/- weight).

For administration the amphiphilic lipids may be formulated into any suitable pharmaceutical composition. The preparation of a dispersion for local administration, such as instillation or injection, wherein intraperitoneal administration is specially preferred.

According to an especially preferred embodiment, the amphiphilic lipids are administered dispersed in an aqueous liquid and the amount of liquid administered is above the minimal coating volume, i.e. above the volume containing sufficient amounts of amphiphilic lipids necessary to coat all internal surfaces. The water will be absorbed by the organs and the amphiphilic lipids will form a coating on the internal surfaces.

In a further aspect the present invention relates to methods for preparing pharmaceutical compositions for treating tumor metastasis, which method comprises steps wherein amphiphilic lipids, which are phospholipids, as the only active compound are mixed with an aqueous solution. The amphiphilic lipids are preferably dispersed in an aqueous solution. The dispersion can be obtained by a number of alternatives well known in the art such as high pressure homogenization. Preferably the dispersion is prepared in a manner such that liposomes with a particle size of 40 to 100 nm are obtained.

The method for preparation of the pharmaceutical composition further preferably also comprises a step, wherein the dispersion or the components used for the preparation thereof are sterilized.

Also in this embodiment of the present invention, the amphiphilic lipid is a phospholipid. Further, the amphiphilic lipids can be a single specific type of amphiphilic lipid or a combination of different amphiphilic lipids. The pharmaceutical composition does not contain any active compounds presently used for the treatment of tumors and metastasis. The amphiphilic lipids are the only active drug compound in the pharmaceutical composition.

### Brief Description of the Figures

- Fig.1: shows the results of analysis of tumor volume in the pre- sence of various concentrations of PL as described in ex- ample 2.
- Fig.2: shows the results of analysis of mean area of tumor at- tachment in the presence of various concentrations of PL as described in example 2.
- Fig.3: shows the results of analysis of the Peritoneal Cancer Index (PCI) in the presence of various concentrations of PL as described in example 2.
- Fig.4: shows the results of the analysis of the relative sur- vival of the test animals used in example 2.
- Fig.5: shows the results of the relative survival of the test animals used in example 3.
- Fig.6: shows the results of analysis of the Peritoneal Cancer Index (PCI) in the presence of various concentrations of PL as described in example 3.
- Fig.7: shows cecum with abrasion of peritoneum a. 30 days after PL150 b. 30 days after NaCl
- Fig.8: shows the results of analysis of tumor volume in the pre- sence of various concentrations of PL as described in ex- ample 3.
- Fig.9: shows the results of analysis of mean area of tumor at- tachment in the presence of various concentrations of PL as described in example 3.
- Fig.10: shows the results of the adhesion assay of example 4, i.e. inhibition of adhesion of rectal cancer cells on microtiter plates coated with In in the presence of va- rious concentrations of PL.
- Fig.11: shows the results of the adhesion assay of example 4, i.e. inhibition of adhesion of rectal cancer cells on microtiter plates coated with coll IV in the presence of various concentrations of PL.
- Fig.12: shows the results of the adhesion assay of example 4, i.e. inhibition of adhesion of gastric cancer cells on microtiter plates coated with fn in the presence of va- rious concentrations of PL.
- Fig.13: shows the results of the adhesion assay of example 5, a) adhesion of gastric cancer cells on laminin in the pre- sence of 1.0 g PL. b) adhesion of gastric cancer cells on laminin in the con- trol group.

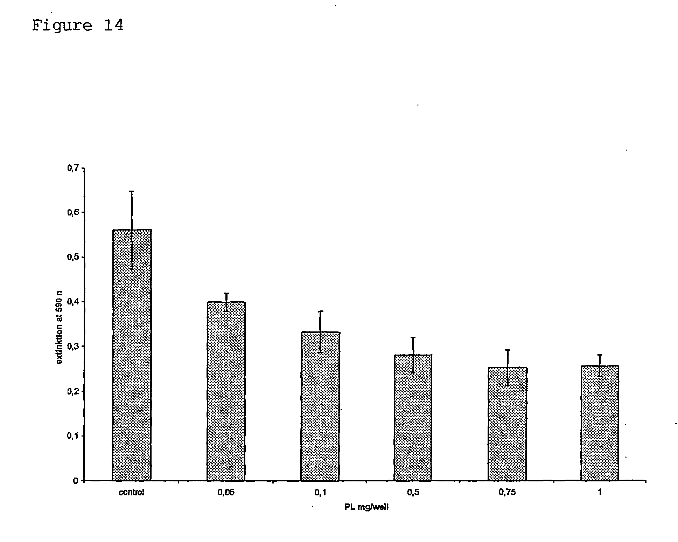

- Fig.14: shows the results of the adhesion assay of example 5, i.e. inhibition of adhesion of gastric cancer cells on microtiter plates coated with In in the presence of va- rious concentrations of PL.
- Fig.15: shows the results of the adhesion assay of example 5, i.e. inhibition of adhesion of gastric cancer cells on microtiter plates coated with fn in the presence of va- rious concentrations of PL.
- Fig.16: shows the results of the adhesion assay of example 5, i.e. inhibition of adhesion of gastric cancer cells on microtiter plates coated with coll IV in the presence of various concentrations of PL.

### Example 1

The method for preparing the PLs may depend on the chosen source of the PLs and the molecules to be obtained. The level of purification can be of particular importance for PLs of natural origin. Purification methods to achieve quality readily acceptable for intravenous use, based on e.g. repeated solvent extraction/precipitation processes, are known to those skilled in the art (F Nielloud, G Marti Mestres, Pharmaceutical Emulsions and Suspensions, Chs 1.VB , 6.VI Marcel Dekker, NY 2000; with further references).

To provide an PLs in a form suitable for the intended purpose, the phospholipids are regularly formulated to a liposomal dispersion which can be manufactured and stored until use. A liposomal dispersion will readily adsorb onto the internal surfaces of the patient, such as the internal surfaces of the peritoneum, and thus form mono- or multilamellar layers which will prevent adhesion of tumor cells to the coated surfaces.

From a pharmaceutical point of view such a liposome dispersion should fulfil several conditions. For stability and functionality reasons, the dispersion should preferably be prepared using high pressure homogenisation or other methods that are suitable for obtaining very small particulate size (sub-micron). In addition, the solution should be sterile. Apropriate sterility assurance levels are reached by thermal sterilisation which is used as the preferred choice for achieving sterility. This puts, however, strains on the physiochemical stability of the formulation.

Optimised formulations containing appropriately balanced phospholipids with good bilayer forming properties, or formulated with additives which could help to provide a stable liposomal dispersion, can redily be prepared using industrial methods for preparation and sterilisation (I Hanin and G Pepeu (eds.): "Phospholipids" p 115-122, Plenum Press NY 1990; M J Groves, "Parenteral Products", Heinemann 1973).

The following phospholipid dispersions were prepared:
1. 30 g egg phospolipids
   25 g glycerol
   NaOH to pH = 8
   Water for injection (WFI) to 1000 ml
2. 20 g lecithin
   NaCl/phosphate buffer to pH = 7 and isotonicity
   WFI to 1000 ml
3. 20 g lecithin (phosphatidylcholin)
   0,1 g natriumoleate
   25 g glycerol
   WFI to 1000 ml
4. 25 g phosphatidylcholin
   5 g phosphatidylethanolamin
   NaCl phosphate buffer to pH = 7 and isotonicity
   WFI to 1000 ml

The PL powder was stirred in the re-cited liquid and subjected to high pressure homogenization until a very fine dispersion of liposomes containing particles having a size in the range of 50-100nm are obtained. The dispersion thus obtained was sterilized by autoclaving and stored until further use.

In some of the following examples the dispersion 1., above, has been used.

### Example 2

Developing peritoneal carcinosis after intraperitoneal injection of tumor cells (cell line: DHD/K12/TRb) into BD IX rats is a well established animal model (Dunnington et al., Int J Cancer 1987; 39:248-254; Reisser et al., Bull Cancer 1991; 78:249-252; and Qin et al., Int J Cancer 1991; 47:765-770) that was used in the present experiment to see if the phospholipids have a therapeutic effect.

### 1. Cell Culture

The cell line DHD/K12/TRb was used. The cells were obtained from a colon adenocarcinoma induced in syngenic BD-IX rats (Garcia-Olmo et al., Cancer Lett 1998; 132:127-133). The cell line was purchased from the European Collection of Animal Cell Cultures (ecacc, Salisbury, UK).

The cells were cultivated in monolayers in a tissue culture flask (75 cm² Falcon, Becton Dicidson-Gambil, Heidelberg, Germany) in DMEM and Ham's F10 (1:1; GIBCO) supplemented with 10% fetal bovine serum (GIBCO) and 0.005% gentamycin (GIBCO). Cell cultures were incubated at 37°C in a humidified atmosphere of 5% C0₂ in air. Cells were passaged after treatment with 0.125% trypsin for 2 min. The cells were pelleted after centrifugation for 10 min at 200g, suspended in 20 ml PBS, and pelleted again. The cell pellet was resuspended in 30 ml complete medium and seeded with a splitting ratio of 1:3. Only cells from three passages were used for the experiments. On the day of Operation 2x10⁶ cells were suspended in 100µl complete medium.

### 2. Animals and Anesthesia

A total of 90 female BD-IX rats (mean body weight 250 +/- 28 g) were included in this study. The rats were kept in protective cages in pairs with unlimited access to water and standard rat chow. The animals were randomly assigned to nine different groups of equal numbers (Table 1). The surgical procedure was performed under sterile conditions and general anesthesia by intramuscular injection of ketamin 100 mg/kg (Ketamin 10%, Sanofi-Ceva, Düsseldorf, Germany) and xylacine 5 mg/kg (Rompun 2%, Bayer, Leverkusen, Germany).

**Table 1**

| | 30 days - peritoneal lesions | 30 days + peritoneal lesions | 90 days |
|---|---|---|---|
| Phospholipids 75 mg/kg (PL75) | 10 | 10 | 10 |
| Phospholipids 150 mg/kg (PL150) | 10 | 10 | 10 |
| NaCl 0,9% | 10 | 10 | 10 |

### 3. Surgical procedure

Tumor cells were injected together with phospholipid solution via a 2 cm midline incision into the peritoneal cavity. In Group 2 additionally an abrasion of parietal and visceral peritoneum in the right flank and at the cecum was performed using a scalpell. (Muller et al., J Surg Res 2001; 96:68-74). Before surgery and at the end of the observation period the body weight (g) was measured in all animals.

### 4. Evaluation of peritoneal carcinosis

After intervals of 30 and 90 days, respectively, the animals were sacrificed by a lethal dose of Isofluran by inhalation. The abdomen was opened by two paramedian incisions for complete exploration. The expanse of peritoneal carcinosis (mm²) was measured using a digitizer board and calculated by custom-made Software on a personal Computer (Treutner KH et al., J Surg Res 1995; 59:764-71). After subtile resection the tumor volume (ml) was measured by water displacement.

Furthermore, the Peritoneal Cancer Index (PCI), as described by Sugarbaker et al. (Sugarbaker PH et al., Cancer Treat Res 1996; 81:149-68), was determined. The PCI was adapted to tumor sizes in rats. The following lesion size scores were used; LS-I: Tumorsize < 2 mm, LS-2: 2.1-5 mm; LS-3: > 5 mm or confluence. The abdominal exploration was carried out by a independent, blinded observer.

Finally, specimens from the abdominal wall, cecum, jejunum, liver, spleen and omentum majus were taken and fixed in formaldehyde solution. Sections with a thickness of 5 µm were stained with hematoxylin and eosin for light microscopy.

### 5. Statistical Analysis

All data are expressed as means +/- Standard error of the mean (SEM). Statistical analysis was performed with a two way ANOVA with contrasts according to pairwise comparison. The level of significance was adjusted according to Bonferroni to alpha/18 = 0,00278. Survival curves were compared using the Log-Rank-Test.

### 6. Results

Performance of the initial surgical procedure was uneventful in all animals. All rats developed peritoneal carcinosis regardless of the treatment. No adverse side effects were noted after injection of phospholipids.

### Body weight

After 30 days, the mean reduction of body weight was 18 ± 9 g in the control group (C). In the PL75 group as well as in the PL150 group the body weight remained nearly constant with differences of 1 ± 2.8 g and -2 ± 5 g, respectively. Those differences were not significant.

### Volume

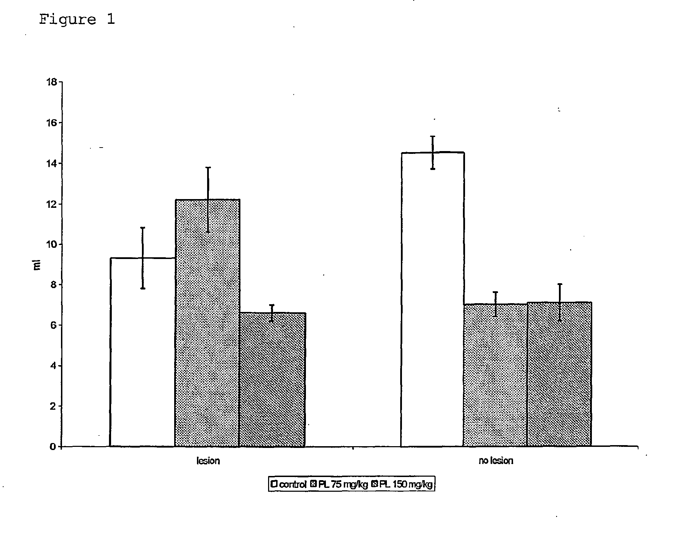

The tumor volume in the control group reached 9.27 ± 1.5 ml with deperitonealisation (C+dp) and 14.45 ± 0.75 ml without peritoneal lesions (C). In the trial group without deperitonealisation, we found a significant reduction of the tumor volume after treatment with both higher phospholipid concentrations to 7 ± 0.59 ml (PL75) and 7.13 ± 0.91 ml (PL150) respectively (p<0.0001). Although a marked reduction of the tumor volume could be found in the PL150+dp, the difference was not significant compared to the control group (p = 0.0817) (Fig.1).

### Area

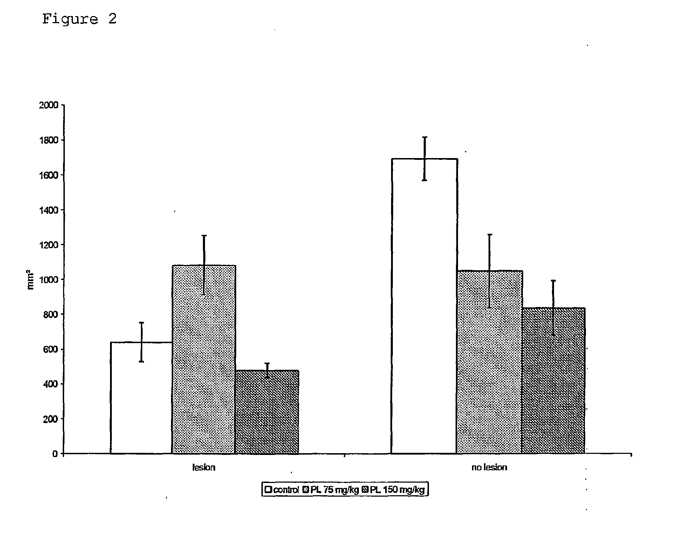

The mean area of tumor attachment in the control group after 30 days was 640 ± 112.5) mm² (C+dp), and 1691 ± 123.6 mm²(C). In the PL75 group we measured 1082.43 ± 169.4 mm² (PL75+dp) and 1048.42 ± 209.3 mm² (PL75). In the PL150 group 480 ± 41.6 mm² (PL150+dp) and 836.49 ± 155.9 mm² (PL150) were proofed. A significant reduction of the area of tumor attachment was found only in the PL 150 group (Fig. 2).

### PCI

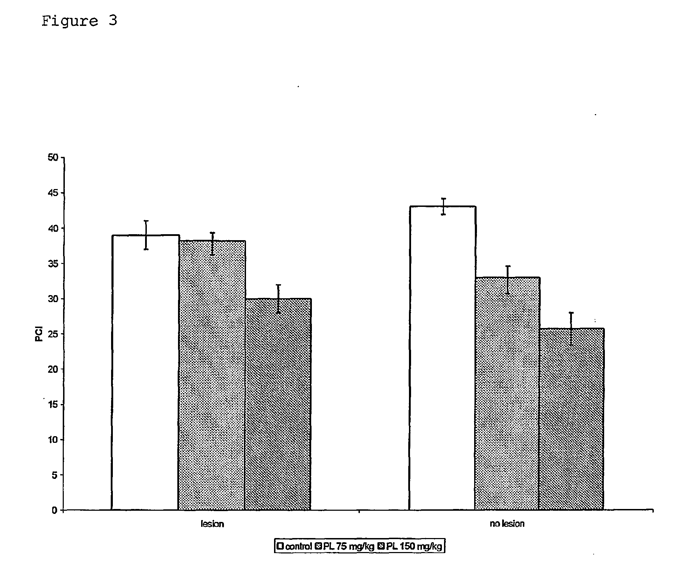

Concerning the Peritoneal Cancer Index (PCI) the control group reached a value of 39 ± 2.03 (C+dp) and 43 ± 1.125 (C), respectively. In comparison to the control group, we found a significant reduction in the PL150 group with 29.6 ± 1.97 (PL150+dp), and 25.7 ± 2.29 (PL150). The PCI registered in the PL75 group was equal in the subgroup with peritoneal lesions (38.2 ± 1.14, PL75+dp) and significantly reduced in the second subgroup (33 ± 1.62, PL75) (Fig. 3).

### Survival rate

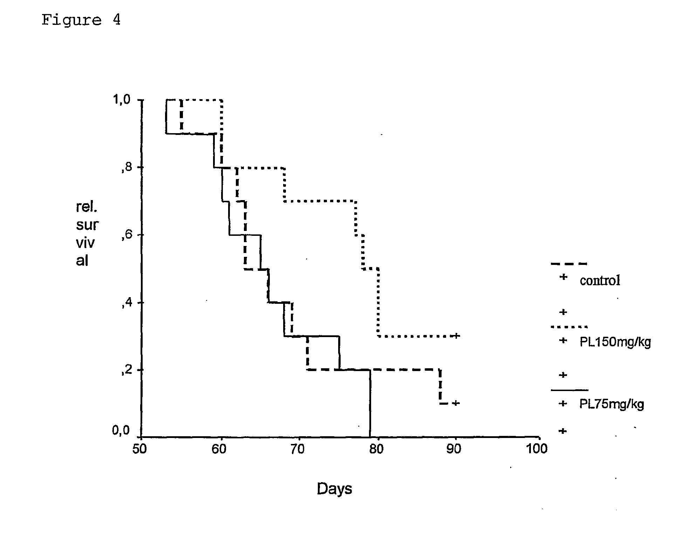

During the 90 day observation period only one animal in the control group survived. The median survival time was 68.5 ± 3.56. All rats in the PL75 group died because of tumor progression until the eighties postoperative day with a median survival time of 66.5 ± 2.79. Survival in the PL150 group (median 74.3 ± 2.71), however, was obviously prolonged compared to the control group although the difference was not significant (p = 0.13). (Fig. 4).

### Histology

Infiltration of colonic cancer could be proofed in all animals histologically. The evidence of tumor infiltration was restricted to the serosa. Invasion into deeper layers could not be detected. A exophytic tumor growth was found. From the histological point no differences occurred in the intraperitoneal tumor growth in the control group compared to the trial groups.

### Example 3

The gastric cancer cell line NUGC-4 was derived from lymph node metastases from a 35 year old female. The histology of the original tumor described a poorly differentiated adenocarcinoma (Schwartz OK, Semin Oncol 1996; 23:316-24; and Akiyama S et al., Jpn J Surg 1988; 18:438-46). The experimental model of peritoneal dissemination in nude mice is well established (Nakashio T et al. Int J Cancer 1997; 70:612-8, Satta T et al., Gastroenterol Jpn 1993; 28:580) and was used in the present study.

### 1. Cell Culture

The human gastric cancer cell line NUGC-4 was purchased from the Japanese Cancer Research Resources Bank (Tokyo, Japan). The cells were maintained in monolayers in a tissue culture flask (75 cm², Falcon, Becton Dikinson-Gambil, Heidelberg, Germany) in RPMI 1640 medium (GIBCO, Paisley, UK), supplemented with 10% fetal bovine serum (GIBCO), penicillin and streptomycin (GIBCO) . Cell cultures were incubated at 37°C in a humidified atmosphere of 5% C0₂ in air. Cells were passaged after treatment with 0.125% trypsin for 6 min. The cells were pelleted after centrifugation for 10 min at 200g, suspended in 20 ml PBS, and pelleted again. The cell pellet was resuspended in 30 ml complete medium and seeded with a splitting ratio of 1:3. Only cells from three passages were used for the experiments. On the day of Operation 1x10⁶ cells were suspended in 100µl complete medium.

### 2. Animals and Anesthesia

A total of 90 female BALB/C nu/nu mice (mean body weight 20.2 +/- 2 g) were included in this study. The animals were kept in protective cages in pairs with unlimited access to water and standard mice chow. The mice were randomly assigned to nine different groups of equal numbers (Table 1). The surgical procedure was performed under sterile conditions and general anesthesia by intramuscular injection of ketamine 40 mg/kg (Ketamin 10%, Sanofi-Ceva, Düsseldorf, Germany) and 0,1 mg/kg KG Medetomidin (Dormitor).

### 3. Surgical Procedure

Tumor cells together with phospholipid solution were injected via a 1 cm midline incision into the peritoneal cavity. In Group 2 additionally an abrasion of parietal and visceral peritoneum in the right flank and at the cecum was performed with a scalpell Before surgery and at the end of the observation period the body weight (g) was measured in all animals.

### 4. Evaluation of Peritoneal Carcinosis

After intervals of 30 and 90 days, respectively, the animals were sacrificed by a lethal dose of Isofluran by inhalation. The abdomen was opened by two paramedian incisions for complete exploration. The expanse of peritoneal carcinosis (mm²) was measured using a digitizer board and calculated by custom-made Software on a personal computer. After subtle resection the tumor volume (ml) was measured by water displacement.

Further, the Peritoneal Cancer Index (PCI), as described by Sugarbaker et al., was determined (Sugarbaker PH et al., Cancer Treat Res 1996; 81:149-68). The PCI was adapted to tumor sizes in mice. The following lesion size scores were used; LS-I: Tumorsize < 2 mm, LS-2: 2.1-5 mm; LS-3: > 5 mm or confluence. The abdominal exploration was carried out by an independent, blinded observer.

Finally, specimens from the abdominal wall, cecum, jejunum, liver, spleen and omentum were taken and fixed in formaldehyde solution. Sections with a thickness of 5 µm were stained with hematoxylin and eosin for light microscopy.

### 5. Statistical Analysis

All data are expressed as means +/- Standard error of the mean (SEM). Statistical analysis was performed with a two way ANOVA with contrasts according to pairwise comparison. The level of significance was adjusted according to Bonferroni to alpha/18 = 0,00278. Survival curves were compared using the Log-Rank-Test.

### 6. Results

All mice developed peritoneal carcinosis, regardless of treatment. No adverse side effects occurred after injection of phospholipids.

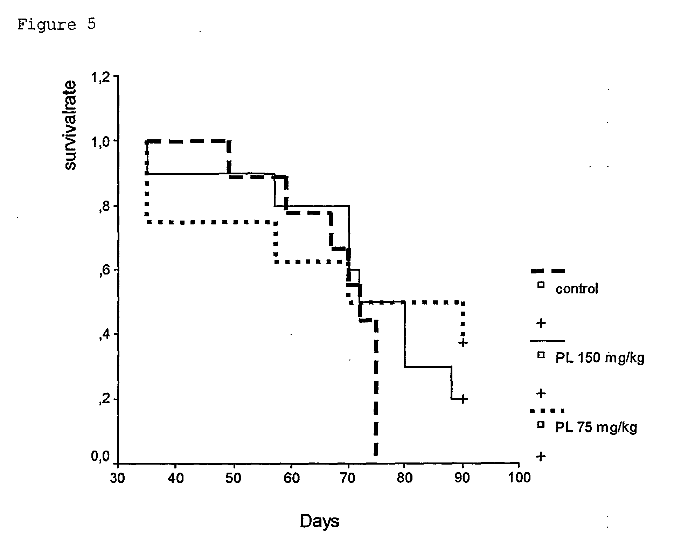

In the control group no mouse lived longer than 75 days. The median survival time was 69 ± 3 days. In the PL150 group the relative survival was longer with a median survival time of 73 ± 5 days. In the PL75 group four animals survived the complete 90 days observation period. However, the median survival time was only 59 ± 6 days (Figure 5).

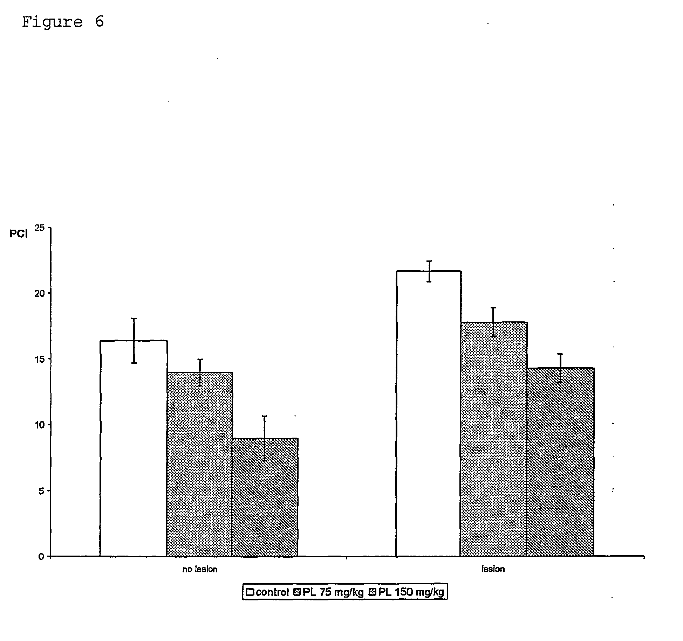

After 30 days the peritoneal cancer index (PCI) in the control group was 16.4 +/- 1.7 without and 21.7 +/- 0.77 with peritoneal lesions (dp). Minor changes occurred after treatment with PL75. The PCI was 14 +/- 1 and 17.8 +/- 1.09 (dp), respectively. However, the differences were not significant compared to the control values. A significant lower PCI was achieved in the PL150 group with 9 +/- 1.68 (p = 0.0002) and 14.3 +/- 1.07 (p = 0.0001) after local deperitonealization (Figure 6).

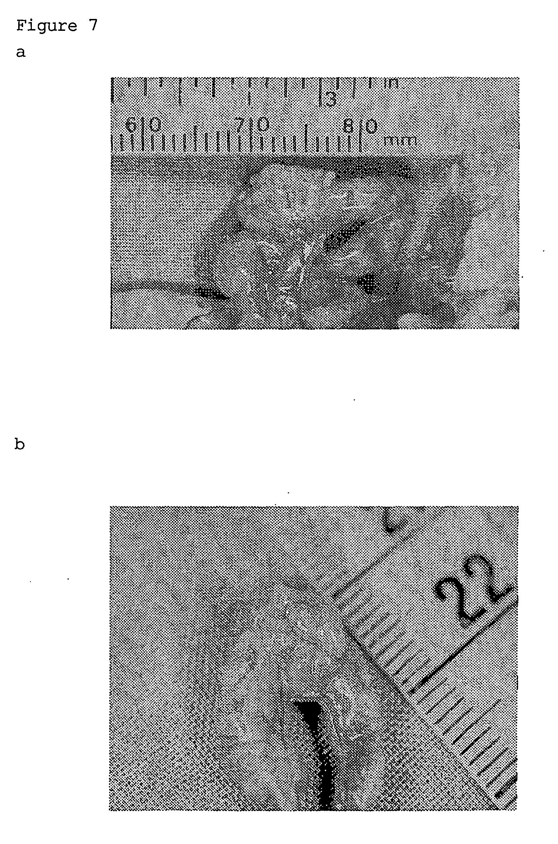

Tumor cell attachment was predominantly found at the site of abdominal incision. If standard peritoneal lesions had been performed, peritoneal carcinosis was focused at these sites (Figure 7 a, b). The mean PCI "Colon" in the control group amounted nearly the maximal value (2.9 +/- 0.09), whereas PCI registered at those sites was reduced in the treatment groups. But, as a consequence of evaluating the complete colon instead of the cecum, we could not find a significant influence of PL on the tumor adhesion compared to the control group in this area. At the PCI "right flank" in both trial groups was decreased significantly. 30 days after treatment the values were 1.7 +/-0.44 in the PL75 group and 0.9 +/- 0.44 in PL150 group, respectively, compared to 2.8 ± 0.13 in the control group.

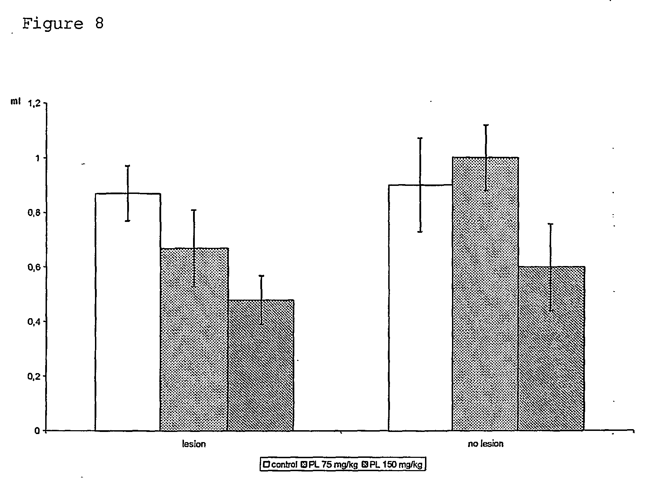

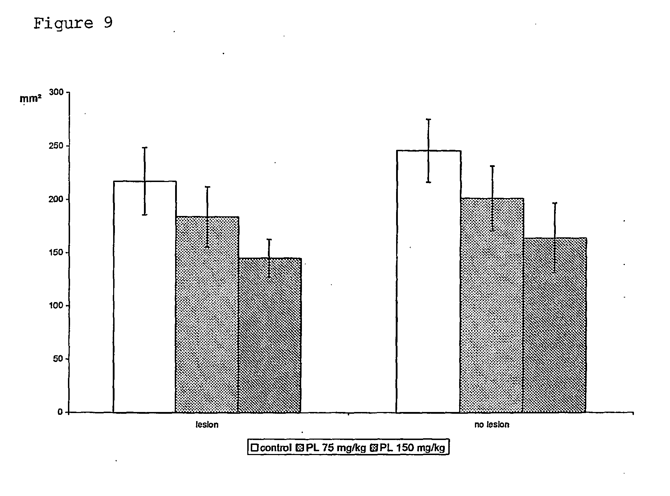

The tumor volume in the control group registered 30 days postoperatively was 0.9 +/- 0.1 ml (dp) and 0.9 +/- 0.17 ml. The respective values in the PL75 group reached 0.67 +/- 0.14 ml (dp) and 1.0 +/- 0.13 ml. Statistical analysis revealed no significant difference in both subgroups. After administration of PL150 the tumor volume in the subgroup with peritoneal lesions could be reduced to a value of 0.48 +/- 0.09 ml (dp) and 0.6 ± 0.16. Both results were not significantly different to those in the control group. However, the p-values were 0.04 (Figure 8). The mean area of tumor cell adhesion in the control group amounted to 217 +/- 31.5 mm² (dp) and 245 +/- 29.3 mm² in comparison with 184 +/- 28.3 mm² (dp) and 201 +/- 30.3 mm² in the PL75 group. The PL150 group developed an respective area of only 145 +/- 17 mm² (dp) and 164 +/- 32.8 mm². The difference between the control group versus PL150 in both groups was not significant, but the p-values were very low with p = 0.049 in the trial without peritoneal lesions and p = 0.08 in the trial with lesions (Figure 9).

These results thus show that PL can be used to inhibit peritoneal tumor cell adhesion especially in the abdominal wall.

### EXAMPLE 4

In this in vitro study the influence of phospholipids on adhesion of colonic cancer cells to extracellular matrix components was determined. Specifically, the reactivity of the cancer cells with several integrins, namely main basement membrane components Collagen IV (coll IV) and laminin (ln) as well as fibronectin (fn), which plays an important role in wound healing, were analyzed.

### 1. Tumor Cells

The human rectal cancer cell line HRT-18 was purchased from the Japanese Cancer Research Resources Bank (Tokyo, Japan). The cells were maintained in monolayers in tissue culture flask (75 cm², Falcon, Becton Dickinson-Gambil, Heidelberg, Germany) in RPMI 1640 medium (GIBCO, Karlsruhe, Germany), supplemented with 10% fetal bovine serum (GIBCO), penicillin and streptomycin (GIBCO). Cell cultures were incubated at 37°C in a humidified atmosphere of 5% CO2 in air. Cells were passaged after treatment with 0.125% trypsin for 6 min. The cells were pelleted after centrifugation for 10 min at 200g, suspended in 20 ml PBS, and pelleted. The cell pellet was resuspended in 30 ml complete medium and seeded with a splitting ratio of 1:3. Only cells from three passages were used for the experiments.

### 2. Extracellular Matrix-Components (ECM)

Flat-bottom polystyrene microtiter plates (Becton Dickinson, Heidelberg, Germany) were coated for adhesion experiments. The purified ECM components were dissolved in PBS with the following concentrations: Collagen IV (coll IV) - 2,5 µg/ml (Biomol, Hamburg, Germany), Fibronektin (fn) - 10 µg/ml (Boehringer, Mannheim, Germany) and Laminin (ln) - 50 µg/ml (Boehringer, Mannheim, Germany). We found these concentrations to be optimal in foregoing dilution series. They were added to the wells and incubated at 4°C for 24 hours (coll IV, fn), or at 37°C for 45 min in a humidified atmosphere of 5% CO2 in air (ln) respectively. Nonspecific protein binding of the coated wells was blocked by adding 1% (w/v) BSA (4°C, 12h) and rinsing the wells with Hepes buffer.

### 3. Adhesion Assay

For adhesion experiments colonic cancer cells were detached with collagenase I (15 min, 37°C, Worthington, Freehold, USA), washed once with RPMI 1640, centrifuged (200 g for 10 min), resuspended in RPMI 1640, and preincubated for 30 min in a humidified atmosphere of 5% CO2 in air (37°C). Seventy thousand tumor cells in 100 µlmedium were seeded in ln and fn coated wells and 30.000 cells in coll IV coated wells according to dilution series. Evaluation of adherent cells was performed using cristal violet staining according to the method described by Aumeilley et al., and Tietze et al.. After the incubation time of 30 min the supernatant with non adherent cells was removed by two washes with warmed RPMI 1640. Attached cells were fixed with 30% (v/v) methanol/ethanol for 15 min at room temperature. Cells were stained with 0.1% (w/v) crystal violet (Sigma, Hamburg, Germany), extensively washed with distilled water, and dried at room temperature. The dye was resuspended with 50 µl of 0.2% (v/v) Triton X-100/well and color yields were then measured in an ELISA reader at 590 nm (Titertek Multiscan Plus MKII, Flow Laboratories GmbH, Meckenheim, Germany). Optical density (OD) showed a linear relationship to the amount of cells between 1 x 10³ and 1 x 10⁵ cells per well, as determined by dilution series.

Control dying of BSA/polystyrene without cells led to minimal Optical Density (OD) values of 0.01-0.07. These values were subtracted from those obtained in the experiments Phospholipids

After complete preparation of the tumor cell suspension the PL solution was added with the following concentrations: 0.1, 0.5, 0.75, 1 and 1.5 mg/100 µl medium. The concentrations used were correlated to our in vivo experiments.

### 4. Statistical analysis

All experiments were performed three times in quadruplicate. The data are expressed as means +/- standard error of the mean (SEM). One way ANOVA with contrasts according to pairwise comparison was used for statistical analysis. The level of significance was adjusted according to Bonferroni to alpha/10 = 0,005.

### 5. Results

### Laminin

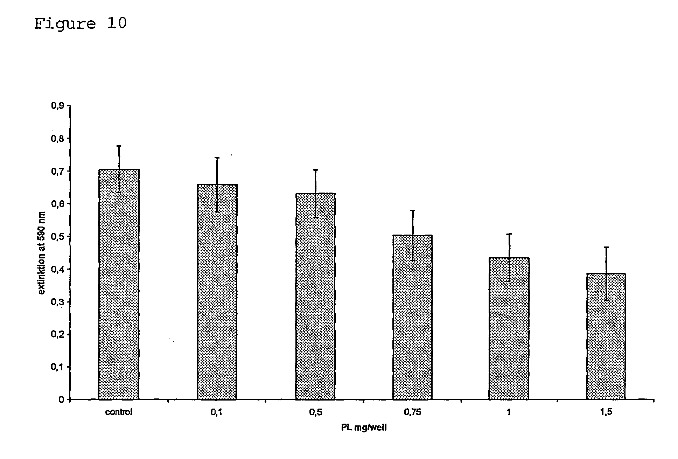

In the adhesionessay of human rectal cells (HRT-18) on laminin after an incubation time of 30 min a mean extinction of 0.7 + 0.07 was detected in the control group. Addition of phospholipids with a concentration of 0.75 (ext. 0.5 ± 0.08), 1 (ext. 0.44 ±0.07) and 1.5 (ext. 0.39 ± 0.08) mg/well led to a significant reduction of tumor cell adhesion (p < 0.0001). Treatment with a concentration of 1.5 mg/well reduced the cell attachment to 46% compared to the control group (Figure 10).

### Collagen IV

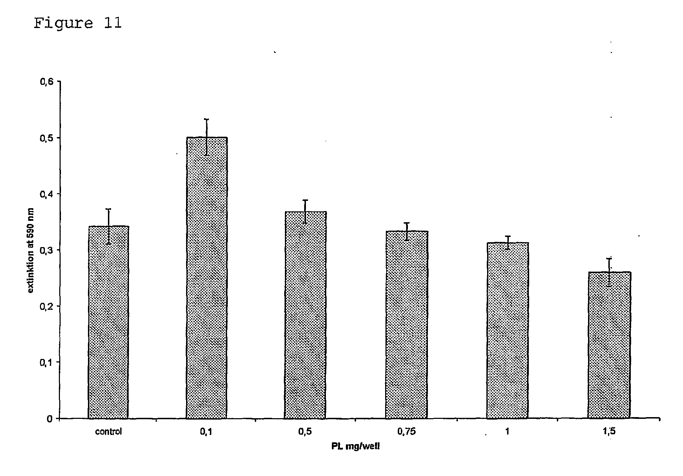

The extinction after incubation of 30.000 tumor cells on collagen IV was 0.34 ± 0.03. While treatment with the lowest concentration of phospholipids (0.1 mg/well) led to an increase of tumor cell adhesion (ext. 0.5 ± 0.03) a reduction of 24 % could be reached with 1.5 mg Phospholipids/well (ext. 0.26 ± 0.02). The differences observed in this trial were compared to the control group were only significant with the maximum concentration (p < 0.0001) (Figure 11).

### Fibronectin

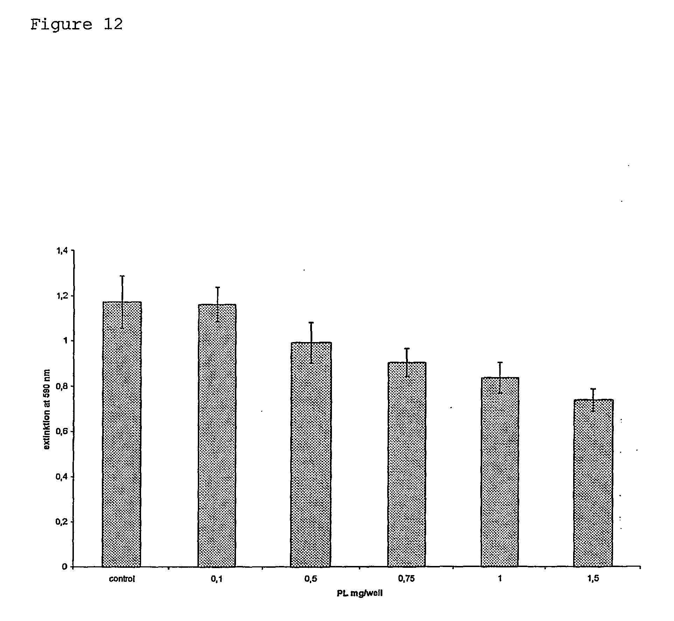

On fibronectin we observed a reduction of tumor cell adhesion analog to the experiments on laminin. Already the treatment with 0.5 mg phospholipids/well (ext. 0.99 ± 0.09) led to a significant reduction of cell attachment compared to the extinction in the control group with a value of 1.17 ± 0.1 (p = 0.0005). The influence of higher phospholipid concentrations (0.75, 1, 1,5 mg/well) was also significant (p < 0.0001). The maximum reduction of tumor cell adhesion on fibronectin was observed after addition of 1.5 mg phospholipids/well (37%) (Figure 12).

### Example 5

In this in vitro study the influence of phospholipids on adhesion of gastric cancer cells to extracellular matrix components was determined.

### 1. Tumor Cells

The human gastric cancer cell line NUGC-4 was purchased from the Japanese Cancer Research Resources Bank (Tokyo, Japan). The cells were maintained in monolayers in tissue culture flasks (75 cm², Falcon, Becton Dickinson-GmbH, Heidelberg, Germany) in RPMI 1640 medium (GIBCO, Karlsruhe, Germany), supplemented with 10% fetal bovine serum (GIBCO), penicillin and streptomycin (GIBCO). Cell cultures were incubated at 37°C in a humidified atmosphere of 5% C0₂ in air. Cells were passaged after treatment with 0.125% trypsin for 6 min. The cells were pelleted after centrifugation for 10 min at 200g, suspended in 20 ml PBS, and pelleted again. The cell pellet was resuspended in 30 ml complete medium and seeded with a splitting ratio of 1:3. Only cells from three passages were used for the experiments.

### 2. Extracellular Matrix (ECM) Components

Flat bottom polystyrene microtiter plates (Becton Dickinson, Heidelberg, Germany) were coated for adhesion experiments. The purified ECM components were dissolved in PBS with the following concentrations: coll IV - 2,5 µg/ml (Biomol, Hamburg, Germany), fn - 10 µg/ml (Boehringer, Mannheim, German), ln - 50 µg/ml (Boehringer, Mannheim, Germany). They were added to the wells and incubated at 4°C for 24 hours (coll IV, fn) or at 37°C for 45 minutes in a humidified atmosphere of 5% CO₂ in air (ln), respectively. Non-specific protein binding of the coated wells was blocked by adding 1% (w/v) BSA (4°C, 12 h) and rinsing the wells with hepes buffer.

### 3. Adhesion assay

For adhesion experiments gastric cancer cells were detached with collagenase I (Worthington, Freehold, USA) for 15 min at 37°C, washed once with RPMI 1640, centrifuged (200 g for 10 min), resuspended in RPMI 1640, and pre-incubated for 30 min in a humidified atmosphere of 5% CO₂ in air at 37°C. 50.000 tumor cells in 100 µl medium were seeded into each well. Evaluation of adherent cells was performed using crystal violet staining according to the method described by Aumeilley et al., and Tietze et al. (Aumailley et al., Exp Cell Res 1989; 181(2):463-474; and Tietze et al., Exp Mol Pathol 1999; 66(2) :131-139). After the incubation time of 30 minutes the supernatent with non-adherent cells was removed by two washes with warmed RPMI 1640.

Attached cells were fixed with 30% (v/v) methanol/ethanol for 15 min at room temperature. Cells were stained with 0.1% (w/v) crystal violet (Sigma, Hamburg, Germany), extensively washed with distilled water, and dried at room temperature. The dye was resuspended with 50 µl of 0.2% (v/v) Triton X-100/well and color yields were then measured in an ELISA reader at 590 nm (Titertek Multiscan Plus MKII, Flow Laboratories GmbH, Meckenheim, Germany). Optical density (OD) showed a linear relationship to the amount of cells between 1x10³ and 5x10⁴ cells per well, as determined by dilution series. Control dying of BSA/polystyrene without cells led to Optical Density (OD) values of 0.01-0.07. These values were subtracted from those obtained in the experiments.

### 4. Phospholipids

After complete preparation of the tumor cell suspension, the phospholipid solution was added with the following concentrations: 0.05, 0.1, 0.5, 0.75, and 1 mg/100 µl medium. The concentrations used were correlated to the subsequent in vivo experiments.

### 5. Statistical analysis

All experiments were performed three times in quadruplicate. The data are expressed as means +/- Standard error of the mean (SEM). One way ANOVA with contrasts according to pairwise comparison was used for statistical analysis. The level of significance was adjusted according to Bonferroni to alpha/10 = 0,005.

### 6. Results

The analysis of tumor cell adhesion to BSA 1% led to a mean extinction of 0.27 ± 0.013 at 590 nm. Coating with laminin (ln) and fibronectin (fn) led to a nearly twofold increase of tumor cell adhesion with mean values of 0.56 ± 0.09 (ln) and 1.17 ± 0.1 (fn), respectively. The cancer cells showed a most pronounced adhesion to collagen IV (coll IV) with a mean extinction of 0.97 ± 0.05.

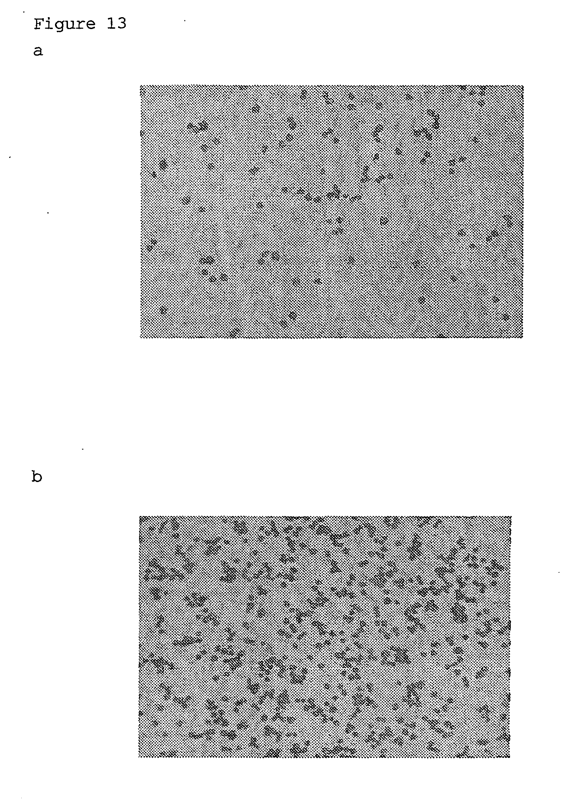

The tumor cell adhesion on ln registered after addition of PL was significantly reduced. The effect was concentration dependent compared to the control. Even the minimal amount of 0.05 mg PL/100µlled to a reduced extinction of 0.4 ± 0,02. Treatment with 0.1 or 0.5 mg PL/100 µl revealed extinction values of 0.33 ± 0.05 and 0.28 ± 0,04, respectively. The maximal effect could be demonstrated with 0.75 mg PL/100µl with an extinction of 0.25 ± 0.04. The relative reduction of tumor cell adhesion on laminin compared to the control amounts 59%. Treatment with 1 mg PL/100µl showed no further decrease of tumor cell adhesion. The mean extinction was 0.26 ± 0.02. Statistical evaluation revealed significant differences for all phospholipid concentrations compared to the control group (p < 0.0001) (Figure 13 a,b; 14);.

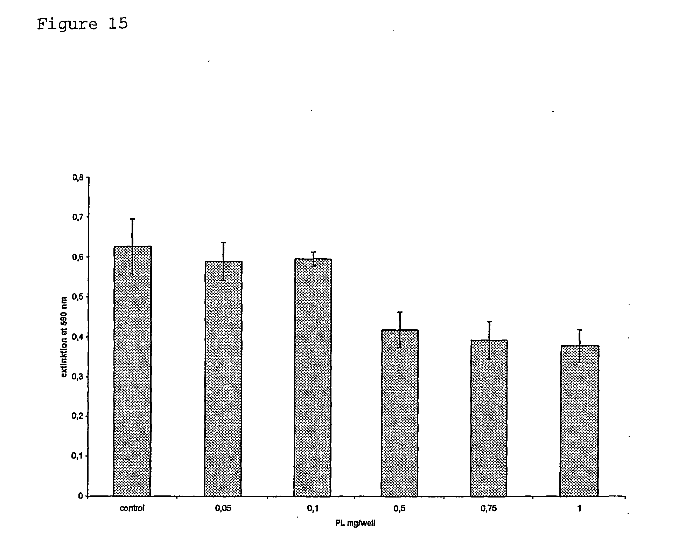

The tumor cell adhesion on fn could not be reduced significantly with low phospholipid concentrations. Addition of 0.05 mg PL/100µl and 0.1 mg PL/100µl resulted in a slightly reduction of the extinction with mean values of 0.59 ± 0.018 and 0.6 ± 0.009. However, a significant reduction of tumor cell adhesion could be observed after treatment with 0.5 mg PL/100µl, (ext. 0.44 ± 0.023), as well as with 0.75 mg PL/100µl, (ext. 0.41 ± 0.025), and 1 mg PL/100µl (ext. 0.39 ± 0.02). We found a similar situation compared to In with equal effects of both 0.75 mg/100µl and 1 mg/100µl PL indicating that the maximum influence on adhesion is reached. The relative reduction of tumor cell adhesion compared to the control values amounts 35% (Figure 15).

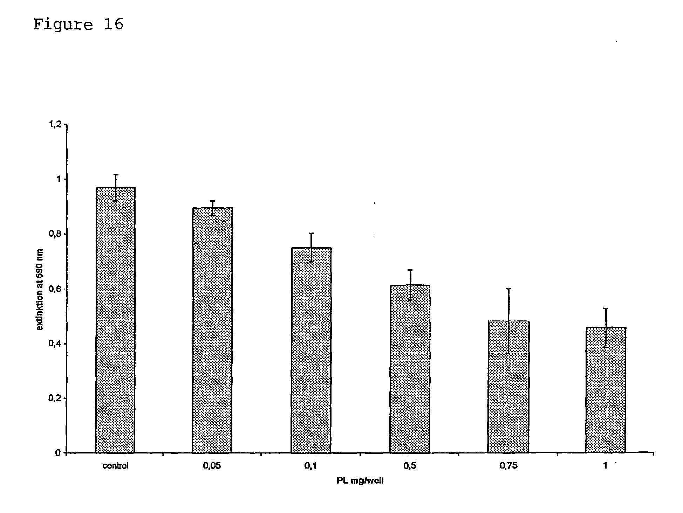

NUGC-4 gastric cancer cells adhere to collagen IV increasingly compared to all other examined extracellularmatrix components. The influence of phospholipids on cell adhesion to coll IV was also concentration dependent. The reduction ranged from a mean extinction of 0.9 ± 0.02 after administration of 0.05 mg PL/100µl to a maximal effect after treatment with 1 mg PL/100µl, with a mean value of 0.46 ± 0.03. Significant differences were found from 0.1 to 1 mg PL/well (p < 0.0001). In comparison to the control value, this means a maximal reduction of adhesive tumor cells of 53% (Figure 16).

In summary, the experimental evidence provided demonstrates that intraperitoneal PLs have an effect in reducing the adhesion of colorectal tumor cells in rats, gastric cancer cells in nuked mice and inhibit the adhesion of gastric cancer cells as well as rectal cancer cells on microtiter plates coated with collagen IV, laminin and fibronectin. These results thus show that PLs inhibit peritoneal tumor cell adhesion and are thus suitable to reduce tumor metastasis.

## Claims

1. Use of amphiphilic lipids as the only active compound for the preparation of a pharmaceutical composition for reducing tumor metastasis, wherein the amphiphilic lipids are phospholipids.

2. Use of amphiphilic lipids according to claim 1, wherein the phospholipid is a phosphoglyceride and/or a phosphatidylcholine.

3. Use of amphiphilic lipids according to claim 1 or 2, wherein a single specific type of amphiphilic lipid or a combination of different amphiphilic lipids are used for the preparation of the pharmaceutical composition.

4. Use of amphiphilic lipids according to claim 1, wherein tumor metastases are reduced due to inhibition of tumor cell adhesion, due to inhibition of adhesion of metastatic cells and/or inhibition of tumor metastasis growth.

5. Use of amphiphilic lipids according to one of claims 1 to 4, wherein tumor metastases are reduced by prophylactic treatment of intraperitoneal recurrence of tumors, specifically malignant tumors, e.g. gastro-intestinal cancer, such as gastric, colon, or pancreatic cancer, and cancer of the genito-urinary system such as ovarian cancer or bladder cancer.

6. Use of amphiphilic lipids according to one of claims 1 to 5, wherein the phospholipid is used for therapeutic or prophylactic treatment of patients.

7. Use of amphiphilic lipids according to claim 6, wherein the patient has undergone curative or palliative resection.

8. Use of amphiphilic lipids according to claims 6 or 7, wherein the patient is a mammal.

9. Use of amphiphilic lipids according to claim 8, wherein the patient is a human.

10. Use of amphiphilic lipids according to one of claims 1 to 9, wherein the amphiphilic lipid is a phospholipid which is present as an aqueous solution containing 0.05% to 25% (w/v) of a phospholipid.

11. Use according to claim 10, wherein the phospholipid is present as an aqueous solution containing 0.5% to 20% (w/v) of a phospholipid.

12. Use according to claim 11, wherein the phospholipid is present as an aqueous solution containing 1.5% to 10% (w/v) of a phospholipid.

13. Use of a phospholipid according to one of claims 10 to 12, wherein the phospholipid is formulated into a solution for local administration, such as instillation or injection, preferably for intraperitoneal administration.

14. Method for preparing a pharmaceutical composition for reducing tumor metastasis, which method comprises steps, wherein amphiphilic lipids as the only active compound are mixed with an aqueous solution, wherein the amphiphilic lipids are phospholipids.

15. Method according to claim 14, wherein the phospholipid is a phosphatidylcholine.

16. Method according to claim 14, wherein the amphiphilic lipids are dispersed in the aqueous solution.

17. Method according to any of claims 14 to 16, wherein the dispersion is obtained by high pressure homogenization.

18. Method according to any of claims 14 to 17, wherein the tumor metastases are reduced due to inhibition of tumor cell adhesion, due to inhibition of adhesion of metastatic cells and/or inhibition of tumor metastasis growth.

19. Method according to any of claims 14 to 18, wherein tumor metastases are reduced by prophylactic treatment of intraperitoneal recurrence of tumors, specifically malignant tumors, e.g. gastro-intestinal cancer, such as gastric, colon, or pancreatic cancer, and cancer of the genito-urinary system such as ovarian cancer.

## Patentansprüche

1. Verwendung von amphiphilen Lipiden als einzig aktive Verbindung zur Herstellung einer pharmazeutischen Zusammensetzung zur Reduktion von Tumormetastasen, wobei die amphiphilen Lipide Phospholipide sind.

2. Verwendung von amphiphilen Lipiden gemäß Anspruch 1, wobei das Phospholipid ein Phosphoglycerid und/oder ein Phosphatidylcholin ist.

3. Verwendung von amphiphilen Lipiden gemäß Anspruch 1 oder 2, wobei eine einzige bestimmte Art von amphiphilem Lipid oder eine Kombination von verschiedenen amphiphilen Lipiden zur Herstellung der pharmazeutischen Zusammensetzung verwendet wird.

4. Verwendung von amphiphilen Lipiden gemäß Anspruch 1, wobei Tumormetastasen durch Hemmung der Anhaftung von Tumorzellen, durch Hemmung der Anhaftung von metastatischen Zellen und/oder durch Hemmung des Wachstums von Tumormetastasen reduziert werden.

5. Verwendung von amphiphilen Lipiden gemäß einem der Ansprüche 1 bis 4, wobei Tumormetastasen durch vorbeugende Behandlung eines erneuten intraperitonealen Auftretens von Tumoren, insbesondere von malignen Tumoren, z.B. gastro-intestinalen Karzinomen, wie z.B. Magen-, Dickdarm- oder Bauchspeicheldrüsenkarzinom, und Karzinomen des genito-urinalen Systems, wie z.B. Ovarialkarzinom oder Blasenkarzinom, reduziert werden.

6. Verwendung von amphiphilen Lipiden gemäß einem der Ansprüche 1 bis 5, wobei das Phospholipid für die therapeutische oder vorbeugende Behandlung von Patienten verwendet wird.

7. Verwendung von amphiphilen Lipiden gemäß Anspruch 6, wobei der Patient einer heilenden oder lindernden Resektion unterworfen wurde.

8. Verwendung von amphiphilen Lipiden gemäß der Ansprüche 6 oder 7, wobei der Patient ein Säugetier ist.

9. Verwendung von amphiphilen Lipiden gemäß Anspruch 8, wobei der Patient ein Mensch ist.

10. Verwendung von amphiphilen Lipiden gemäß einem der Ansprüche 1 bis 9, wobei das amphiphile Lipid ein Phospholipid ist, welches als wässrige Lösung vorliegt, die 0,05% bis 25% (Gewicht/Volumen) eines Phospholipids enthält.

11. Verwendung gemäß Anspruch 10, wobei das Phospholipid als wässrige Lösung vorliegt, die 0,5% bis 20% (Gewicht/Volumen) eines Phospholipids enthält.

12. Verwendung gemäß Anspruch 11, wobei das Phospholipid als wässrige Lösung vorliegt, die 1,5% bis 10% (Gewicht/Volumen) eines Phospholipids enthält.

13. Verwendung eines Phospholipids gemäß einem der Ansprüche 10 bis 12, wobei das Phospholipid zu einer Lösung zur lokalen Verabreichung, wie z.B. zur Einträufelung oder zur Injektion, vorzugsweise zur intraperitonealen Verabreichung, formuliert ist.

14. Verfahren zur Herstellung einer pharmazeutischen Zusammensetzung zur Reduktion von Tumormetastasen, wobei das Verfahren Schritte umfasst, bei denen man amphiphile Lipide als einzig aktive Verbindung mit einer wässrigen Lösung mischt, wobei die amphiphilen Lipide Phospholipide sind.

15. Verfahren gemäß Anspruch 14, wobei das Phospholipid ein Phosphatidylcholin ist.

16. Verfahren gemäß Anspruch 14, wobei die amphiphilen Lipide in der wässrigen Lösung dispergiert sind.

17. Verfahren gemäß einem der Ansprüche 14 bis 16, wobei die Dispersion durch Hochdruck-Homogenisierung erhalten wird.

18. Verfahren gemäß einem der Ansprüche 14 bis 17, wobei die Tumormetastasen durch Hemmung der Anhaftung von Tumorzellen, durch Hemmung der Anhaftung von metastatischen Zellen und/oder durch Hemmung des Wachstums von Tumormetastasen reduziert werden.

19. Verfahren gemäß einem der Ansprüche 14 bis 18, wobei Tumormetastasen durch vorbeugende Behandlung eines erneuten intraperitonealen Auftretens von Tumoren, insbesondere von malignen Tumoren, z.B. gastro-intestinalen Karzinomen, wie z.B. Magen-, Dickdarm- oder Bauchspeicheldrüsenkarzinom, und Karzinomen des genito-urinalen Systems, wie z.B. Ovarialkarzinom, reduziert werden.

## Revendications

1. Utilisation de lipides amphiphiles en tant que seul composé actif pour la préparation d'une composition pharmaceutique pour réduire une métastase tumorale, où les lipides amphiphiles sont des phospholipides.

2. Utilisation de lipides amphiphiles selon la revendication 1, où le phospholipide est un phosphoglycéride et/ou une phosphatidylcholine.

3. Utilisation de lipides amphiphiles selon la revendication 1 ou 2, où un seul type spécifique de lipides amphiphiles ou une combinaison de différents lipides amphiphiles sont utilisés pour la préparation de la composition pharmaceutique.

4. Utilisation de lipides amphiphiles selon la revendication 1, où les métastases tumorales sont réduites en raison de l'inhibition de l'adhésion des cellules tumorales, en raison de l'inhibition de l'adhésion des cellules métastatiques et/ou de l'inhibition de la croissance des métastases tumorales.

5. Utilisation de lipides amphiphiles selon l'une des revendications 1 à 4, où les métastases tumorales sont réduites par un traitement prophylactique de la réapparition intrapéritonéale des tumeurs, spécifiquement des tumeurs malignes, par exemple un cancer gastro-intestinal, tel qu'un cancer de l'estomac, du côlon, ou du pancréas, et un cancer du système génito-urinaire tel qu'un cancer des ovaires ou un cancer de la vessie.

6. Utilisation de lipides amphiphiles selon l'une des revendications 1 à 5, où le phospholipide est utilisé pour le traitement thérapeutique ou prophylactique des patients.

7. Utilisation de lipides amphiphiles selon la revendication 6, où le patient a subi une chirurgie curative ou palliative.

8. Utilisation de lipides amphiphiles selon la revendication 6 ou 7, où le patient est un mammifère.

9. Utilisation de lipides amphiphiles selon la revendication 8, où le patient est un humain.

10. Utilisation de lipides amphiphiles selon l'une des revendications 1 à 9, où le lipide amphiphile est un phospholipide qui est présent sous forme d'une solution aqueuse contenant 0,05 % à 25 % (p/v) d'un phospholipide.

11. Utilisation selon la revendication 10, où le phospholipide est présent sous forme d'une solution aqueuse contenant 0,5 % à 20 % (p/v) d'un phospholipide.

12. Utilisation selon la revendication 11, où le phospholipide est présent sous forme d'une solution aqueuse contenant 1,5 % à 10 % (p/v) d'un phospholipide.

13. Utilisation d'un phospholipide selon l'une des revendications 10 à 12, où le phospholipide est formulé en une solution destinée à une administration locale, telle Qu'une instillation ou une injection, de préférence pour une administration intrapéritonéale.

14. Procédé de préparation d'une composition pharmaceutique pour réduire une métastase tumorale, lequel procédé comprend des étapes, où des lipides amphiphiles en tant que seul composé actif sont mélangés avec une solution aqueuse, où les lipides amphiphiles sont des phospholipides.

15. Procédé selon la revendication 14, où le phospholipide est une phosphatidylcholine.

16. Procédé selon la revendication 14, où les lipides amphiphiles sont dispersés dans une solution aqueuse.

17. Procédé selon l'une quelconque des revendications 14 à 16, où la dispersion est obtenue par homogénéisation à haute pression.

18. Procédé selon l'une quelconque des revendications 14 à 17, où les métastases tumorales sont réduites en raison de l'inhibition de l'adhésion des cellules tumorales, en raison de l'inhibition de l'adhésion des cellules métastatiques et/ou de l'inhibition de la croissance des métastases tumorales.

19. Procédé selon l'une quelconque des revendications 14 à 18, où les métastases tumorales sont réduites par un traitement prophylactique de la réapparition intrapéritonéale des tumeurs, spécifiquement des tumeurs malignes, par exemple un cancer gastro-intestinal, tel que le cancer de l'estomac, du côlon ou du pancréas, et un cancer du système génito-urinaire tel que le cancer des ovaires.
